# EUROPEAN PATENT SPECIFICATION

(11) **EP 2 591 358 B1**
(45) Date of publication and mention of the grant of the patent: **07.09.2016**
(21) Application number: 11743360.7
(22) Date of filing: 04.07.2011
(51) Int. Cl.: G01N 33/53

(54) **EXAMINATION SYSTEM WITH SAMPLE INCUBATION**
UNTERSUCHUNGSSYSTEM MIT PROBENINKUBATION
SYSTÈME D'EXAMEN À INCUBATION D'ÉCHANTILLON

(30) Priority: 05.07.2010 EP 10168389
(43) Date of publication of application: 15.05.2013
(73) Proprietor: Koninklijke Philips N.V., 5656 AE Eindhoven (NL)
(72) Inventor: HARDEMAN, Wilhelmina, Maria, NL-5656 AE Eindhoven (NL); EVERS, Toon, Hendrik, NL-5656 AE Eindhoven (NL); JANSEN, Theodorus, Petrus, Henricus, Gerardus, NL-5656 AE Eindhoven (NL)
(74) Representative: van Velzen, Maaike Mathilde
(86) International application number: PCT/IB2011/052945
(87) International publication number: WO 2012/004723

(56) References cited:
- WO-A1-2009/060357
- WO-A1-2009/066236
- WO-A1-2009/115951
- US-A1- 2007 144 976
- US-B1- 6 736 978

## Description

### FIELD OF THE INVENTION

The invention relates to apparatus and methods for the examination of a sample, said examination comprising the incubation of the sample with an agent.

### BACKGROUND OF THE INVENTION

From the WO 2009/115951 A1, a cartridge for a biosensor is known comprising a sample chamber that can be filled with a sample to be examined. After the filling, magnetic particles that are stored in reservoirs of the sample chamber are dispersed in the liquid sample and bind specifically to target components in the sample. The labeled target components can then be detected by frustrated total internal reflection at a sensing surface of the chamber.

### SUMMARY OF THE INVENTION

Based on this situation it was an object of the present invention to provide means for the examination of a sample fluid that are particularly suited for an application outside the laboratory, for example in a roadside drug testing or bedside clinical testing. To this end, it is particularly desirable that the means allow for a quick yet accurate examination.

This object is achieved by a method according to claim 1 and an apparatus according to claim 8. Preferred embodiments of the invention are disclosed in the dependent claims.

According to the invention, the invention relates to an apparatus for the examination of a sample, particularly a fluid, for example blood, saliva, or urine. The apparatus comprises a) a cartridge.

The term "cartridge" shall denote in this context a comparatively small container for a sample in which examinations executed by a separate sensor device can take place. As the cartridge is contaminated by the sample, it will usually be a disposable device, produced for example from plastic by injection moulding. The cartridge shall comprise the following components:
i) An inlet where a sample to be examined can be introduced. The inlet may for example be a funnel-shaped hole to which droplets of the sample can be applied by a user.
ii) A reaction chamber where the sample can be examined. The reaction chamber is a cavity that is large enough to contain a portion of the sample and to allow its examination. It may be an empty cavity or a cavity filled with some substance like a gel that may absorb a sample substance. In case of optical examinations, at least one surface of the reaction chamber will typically be transparent. Moreover, the cartridge may integrally comprise some sensors, for example magneto-resistive sensors of the kind described in the WO 2005/010543 A1 or WO 2005/010542 A2.
iii) A channel connecting the inlet with the aforementioned reaction chamber. The channel may in general have any three-dimensional shape, though it will typically be straight and elongated. The cross section of the channel will preferably be smaller than the cross-section of the reaction chamber, as the channel shall only provide for a quick transport of sample but not retain large volumes thereof. Optionally, the channel may comprise means for an active transport of fluid towards the reaction chamber, for example microfluidic pumps.
iv) A reservoir for an agent with which the sample shall be incubated, wherein said reservoir shall be located at the inlet. In this context, the location "at the inlet" shall denote a position that is closer to the inlet than to the reaction chamber (or, more precisely, closer to the nearest point of the inlet than to the nearest point of the reaction chamber). The reservoir may in general be located at any position from the inlet to halfway down the channel. Most preferably, it will be located as close as possible to the inlet, for example within the inlet itself or within the mouth of the channel that connects to the inlet.

In general, the reservoir of the cartridge shall only be capable to retain an agent, particularly a solid agent that is soluble in the sample. In a preferred embodiment, the reservoir shall already be pre-filled with such an agent, i.e. the cartridge leaves the factory ready-to-use.

The described cartridge enables the examination of a sample without special laboratory equipment because it has a separate inlet that can be designed optimally with respect to a convenient manual introduction of the sample, for example from a syringe or pipette. The cartridge further has a separate reaction chamber where optimal conditions for the desired examination of the sample prevail, said examination comprising the incubation of the sample with an agent. The described design inevitably implies that there will be some time needed for a sample to be transferred from the inlet to the reaction chamber, which adds to the duration of the total examination procedure. The examination time should however be as short as possible for an out-of-laboratory procedure. The described cartridge turns this apparent drawback into an advantage by arranging the reservoir for the agent at the inlet. This has the consequence that dispersion of the agent in the sample and hence incubation of the sample can start immediately during the introduction of the sample into the cartridge. The time needed for transporting the sample from the inlet to the reaction chamber is therefore not lost but additionally used for the incubation of the sample.

It was already mentioned that the filling of the reaction chamber with a sample takes some time which adds to the total duration of an examination. The filling-course typically depends on the geometry of the cartridge and the consistency of the sample. As the filling duration usually has an important influence on the outcome of an examination, the above determination of the filling-course provides valuable additional information about the examination procedure and its boundary conditions. This can for instance be used to estimate the reliability of an examination and to discard results obtained under abnormal filling-courses.

In the context of the present invention, effective filling times (measured from the beginning of sample introduction at the inlet) are typically longer than 20% of the total examination time, often longer than 50% of the total examination time (corresponding typically to absolute values from about 10 seconds to more than about one minute).

According to the invention, the apparatus further comprises:
b) A filling detector for determining the filling-course of the cartridge and/or of the reaction chamber, wherein the filling-course is defined as above.

With the aforementioned apparatus, the above described determination of the filling-courses can be realized, thus allowing to improve the accuracy of the examination results.

The agent that it is applied to the sample may comprise any component that shall assist the intended examination. In many important examples, the agent comprises label particles that specifically bind to target components in the sample. In this context, the term "label particle" shall denote a particle (atom, molecule, complex, nanoparticle, microparticle etc.) that has some property (e.g. optical density, magnetic susceptibility, electrical charge, fluorescence, radioactivity, etc.) which can be detected, thus indirectly revealing the presence of the associated target component. The label particles may particularly be magnetic particles, for example superparamagnetic beads. This allows to actuate said particles (and the bound target components) by appropriate magnetic fields and/or to detect the labeled target components via a magnetic moment of their labels.

In a further development of the cartridge and/or of the method described above, the cartridge comprises a filter through which the sample can be passed on its way from the inlet to the reaction chamber. Filtering of raw samples is usually needed to avoid disturbances of the examination by contaminations or dirt.

The determination of the filling-course can for instance simply be used to determine a reliability of the examination results, considering for example results as invalid if the effective filling time is out of some given range. In more elaborate approaches, the examination procedure may be adapted according to the determined filling-course. The start of measurements may for example be delayed or advanced in order to achieve an approximately constant incubation time, or the sample in the reaction chamber may be actuated more or less intensely.

In practice it turns out that the filling-course - and hence the incubation time - varies considerably from sample to sample due to different (viscous) properties of the samples. As the outcome of the examination usually depends significantly on the incubation time, the examination results will therefore vary from sample to sample, too. In a preferred embodiment of the invention, the effective incubation of the sample with an agent is determined from the filling-course, particularly from a measured spatiotemporal course of filling of the cartridge or the reaction chamber. In this context, the term "effective incubation" shall refer to the fact that different parts of the agent will usually come into contact with a sample at different times and will therefore experience different individual incubation times. The "effective incubation" may take this into account. It may for example comprise an average value of the individual incubation times of all parts of the agent.

According to the invention, the evaluation of the examination results is adapted according to the determined filling-course. Referring to the aforementioned embodiment, the examination results may particularly be corrected according to their dependence on the effective incubation of the sample with the agent. This approach is possible if the dependence of the examination results on the incubation time is (approximately) known. An advantage of this approach is that it can primarily be realized in software. In the apparatus, the control and/or evaluation modules have to be designed such that they are capable to execute the described adaptations.

The filling detector of the apparatus according to the invention can be realized in a variety of ways. In a first realization, the filling detector comprises an imaging device (e.g. a CCD chip) and an image processing device (e.g. a microcomputer with appropriate software) for detecting a fluid meniscus in the reaction chamber. An imaging device like a camera is in many applications already available for the optical observation of processes in the reaction chamber. Said device can then additionally be used to detect the fluid meniscus of a sample filling the reaction chamber, which allows to determine the filling-course.

In another example, the filling detector is adapted to detect the arrival of sample fluid at a plurality of investigation regions in the reaction chamber. In many applications, such a plurality of investigation regions is provided in the reaction chamber to allow for a plurality of parallel investigations, for example with different binding sites. The filling detector only needs to detect in a binary sense if sample fluid is present in an investigation region or not. The distribution of investigation regions over a surface of the reaction chamber then provides a spatial picture of the inflow of sample into the chamber. It should be noted that the aforementioned imaging approach can be seen as a special example of this method, if the pixels of the image are interpreted as investigation regions.

In a further example, the filling detector is adapted to process image data of a plurality of investigation regions. This embodiment is based on the definition of a plurality of particular regions in the reaction chamber, the "investigation regions" or "regions of interest", and uses image data from these investigation regions. These image data are then processed, for example in order to detect the arrival of sample fluid in an investigation region at hand. In general, the processing may relate to many other events or characteristics that shall be detected, for example to the dissolution of a dissolvable layer on the observed surface of the reaction chamber (wherein this dissolution may take place later than the arrival of a fluid meniscus). The image data of the investigation regions will typically be sets of image values (e.g. gray values) at pixels representing the investigation regions. In practice, a complete examination area of the reaction chamber will typically be represented in a single image generated by an image sensor (camera), and the investigation regions will be different sub-regions defined within this overall image.

In the aforementioned example, the processing of the image data of the investigation regions may particularly be adapted to detect transitional and/or permanent changes of the image data occurring during an assay (or, more specifically, occurring during the filling time of the cartridge or reaction chamber). Moreover, the processing may particularly comprise the temporal and/or spatial filtering of image values for single investigation regions and/or for several (or all) investigation regions. For example, the pixel values of one investigation region may be spatially averaged to yield a single image datum for this investigation region. Furthermore, the pixel values or the aforementioned averaged image data of an investigation region may be temporarily filtered, for example in order to remove artifacts like changes caused by accidental movements of the cartridge with respect to the sensor device.

According to another example, the filling detector comprises at least one wetting detection site within the cartridge. This wetting detection site may particularly be located at some distant position within the reaction chamber, which is reached not before the remainder of the reaction chamber is already filled with sample. Another possible location of a wetting detection site is at the inlet; this position would allow to exactly determine the start of the effective filling time.

The invention further relates to the use of the apparatus described above for molecular diagnostics, biological sample analysis, chemical sample analysis, food analysis, and/or forensic analysis. Molecular diagnostics may for example be accomplished with the help of magnetic beads or fluorescent particles that are directly or indirectly attached to target molecules.

### BRIEF DESCRIPTION OF THE DRAWINGS

These and other aspects of the invention will be apparent from and elucidated with reference to the embodiment(s) described hereinafter. These embodiments will be described by way of example with the help of the accompanying drawings in which:
Figure 1 schematically shows a sectional side view of an examination apparatus with a cartridge according to the present invention;
Figure 2 is a diagram illustrating the kinetics of target binding to magnetic beads;
Figure 3 shows measured optical FTIR signals for a plurality of investigation regions in a reaction chamber;
Figure 4 shows in a top view the uneven filling of a reaction chamber with a sample;
Figure 5 shows measured optical FTIR signals for a plurality of investigation regions in a reaction chamber in which the examination surface is initially covered with a sucrose layer;
Figure 6 shows images of a reaction chamber with an enlargement of one ROI before (left) and after (right) dissolution of a sucrose layer;
Figure 7 shows corresponding measurement data for several investigation regions when wetting occurs (left) or does not occur (right);
Figure 8 shows an exemplary processing scheme for image data of two investigation regions.

Like reference numbers in the Figures refer to identical or similar components.

### DESCRIPTION OF PREFERRED EMBODIMENTS

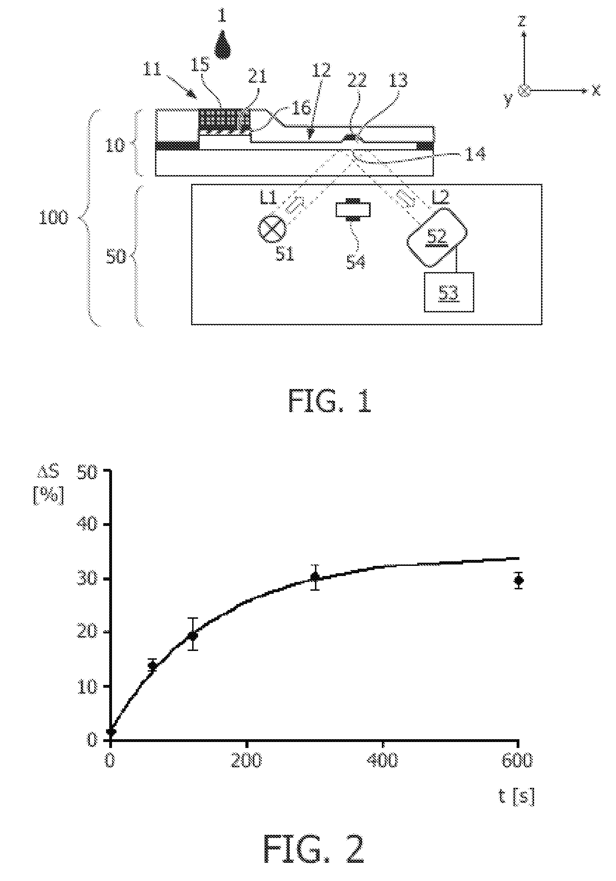

An examination apparatus 100 according to the present invention is schematically shown in Figure 1. This apparatus 100 comprises two basic components, namely a cartridge 10 and a sensor device 50.

The cartridge 10 is typically a disposable device made for example from glass or from (transparent) plastic by injection moulding. The main function of the cartridge 10 is to provide a container in which examinations of a sample 1, for example a droplet of blood, can take place. To this end, the cartridge 10 comprises the following microfluidic components:
- An inlet 11 where the sample 1 can be introduced, for example by applying it from a pipette. The inlet is optionally provided with a filter 15 for filtering an introduced sample.
- A channel 12 that is connected at its mouth to the inlet 11.
- A reaction chamber 13 that is connected to the other end of the channel 12 and that provides a space where the sample can be examined. The top of the reaction chamber 13 can optionally be provided with some agents 22 that are solved in the sample once it is in the reaction chamber 13.

In the shown example, the bottom of the reaction chamber 13 provides at least one investigation region 14 at which sensing of the sample can take place. As will now be explained in more detail, this sensing is executed by the sensor device 50 according to the principle of frustrated total internal refraction (FTIR).

The sensor device 50 is located at the bottom side of the cartridge 10. It comprises means for the detection of target components, particularly target components labeled with magnetic particles (e.g. superparamagnetic beads), that are specifically bound to binding sites in the investigation region 14. The sensor device 50 comprises a light source 51 for emitting an input light beam L1 towards the interface between cartridge 10 and sample in the investigation region 14, where it is totally internally reflected into an output light beam L2. Target components and/or magnetic particles that are bound at the interface will lead to a frustrated total internal reflection (FTIR), which can be detected in the output light beam L2 with the help of a light detector 52. The light detector 52 may particularly be an imaging device like a CCD or CMOS camera. The measurement signals of this detector 52 are evaluated and recorded by an evaluation module 53. Moreover, the Figure shows a magnetic field generator 54 disposed below the cartridge 10 for generating a magnetic actuation field for manipulating magnetic particles inside the reaction chamber 13. More details about the measurement principle of FTIR may for example be found in the WO 2008/155716, WO 2009/001276 A1, and WO 2008/142492 A1.

In practice, cartridges like the one shown in Figure 1 will be used in a point-of-care situation where the examination is performed either directly at the roadside, the bedside, or at the physician's office or even at home. Such a point of care test needs to have a fast total assay time (sample in, result out in a typical total assay time of five minutes).

However, to perform a test in a fully integrated cartridge, a number of process steps have to be implemented, for example filtering, filling, redispersion of beads, incubation of target, binding to the sensor surface, washing, detection. Depending on the fluidic properties of the (plasma) sample, filling of the cartridge can take up to one minute. Also the redispersion of the beads and incubation with the target molecules takes typically 1 to 1.5 minutes. Before the surface reaction (which is considered a rate-limiting step) can start, already as much as half the assay time can be already spent.

A second problem arises due to the variation in biological samples. Biological samples from different people will show big variety in fluidic behavior, amount of cells, etc. Therefore the filling time will be different for every cartridge/sample. Typical experiments have shown a spread of filling times between less than 1 sec and 44 sec. If the beads used in the assay are provided dried inside the reaction chamber (e.g. as agent 22 in Figure 1), the incubation step starts when the beads redisperse as soon as they come in contact with the sample liquid. The incubation step is important because it determines the amount of target molecules, which have bound to the beads and therefore the total amount of binding to the sensor surface in the end. A difference in the time it takes to fill the reaction chamber can therefore have an influence on the amount of target detected, resulting in an imprecision in the assay result.

The following table illustrates that the variation in incubation time, due to variation in filling speed leads to different assay results. A solution containing cardiac troponin I (cTnI) was injected into a cartridge containing dry magnetic particles in the reaction chamber, and the incubation step in the actuation was varied between 0 s, 30 s and 60 s. At the end of the reaction of the beads with the surface, the signal change was measured.

| incubation time (sec) | measured assay signal change (%) | standard deviation on signal change (%) |
|---|---|---|
| 0 | 8.9 | 2.4 |
| 30 | 14.4 | 2.7 |
| 60 | 21.1 | 0.8 |

Furthermore, Figure 2 illustrates the kinetics of target binding to beads. 100 pM of cardiac troponin I (cTnI) was incubated with 1 mg/mL beads for different times t (horizontal axis). The excess of unbound cTnI was removed and the beads were injected into a (conventional) cartridge. At the end of the reaction of the beads with the surface, the signal change ΔS was measured (vertical axis).

It should be noted that the degree of the second problem is dependent on the kinetics of the target binding to the magnetic beads. If the assay includes an incubation step after complete filling of the chamber that is longer than the time needed to bind all the target (where the curve in Figure 2 is leveled off), there will of course be no difference between samples with different filling times. However, this limits the freedom in assay design and for many assays this might not be possible within the desired total assay time of 5 minutes.

As a solution to speed up the total assay time, it is proposed to combine different process steps. In particular, it is proposed that the magnetic beads are applied at the beginning of the fluidic channel.

In the embodiment shown in Figure 1, this is achieved by providing a reservoir 16 at the connection between the channel 12 and the inlet 11, wherein this reservoir 16 is pre-filled with e.g. superparamagnetic beads 21 as an agent, i.e. the agent 21 is disposed underneath or right after the filter 15. The advantage of this is that when the sample 1 is applied, the beads 21 will redisperse and the sample, containing the beads, will flow into the cartridge towards the reaction chamber 13 and the incubation has already started. This means that several process steps are combined in one step, which lowers the total assay time. The combined process steps include: filtering of the sample, redispersion of the beads, filling the cartridge and incubation of the target molecules to the beads. This saves time, and the total assay time will be decreased.

The aforementioned approach may however increase the error made due to the differences in filling speed described above, as the incubation time will now be directly related to the filling time of the whole cartridge, rather than the filling time of only the reaction chamber. An appropriate mechanism to correct for these variations is therefore desirable. As a preferred embodiment it is therefore proposed here to measure the "effective filling time" and/or the incubation time and apply some correction for incubation time. In this context, the "effective filling time" is defined as the duration from a predetermined starting point to a predetermined end point of the filling. In practice, said end point is usually reached as soon as the whole reaction chamber is filled with the sample.

A first important definition of the starting point is the point in time at which the introduction of a sample at the inlet begins. If it is not stated otherwise, this definition will be assumed in the following.

Another important definition of the starting point is the point in time at which sample first enters the reaction chamber. Depending on practical considerations, i.e. the measurability of certain events, other definitions of the starting (or end) point can be applied to.

A correction for effective filling time or incubation time could be done in different ways:
- Mathematical correction for the effect of incubation time for the end result (this requires prior knowledge on how the assay results are dependent on the incubation time, cf. the table above for an example).
- Adjustment of actuation scheme to effective filling time, for example applying stronger or softer actuation, or longer or shorter incubation/actuation.

A measurement of the incubation time can be performed in different ways, of which a few are mentioned below:
If the beads 21 are placed directly under the filter 15, wetting of the filter (and therefore the start of the redispersion of the beads) can be detected by an appropriate wetting detector. This embodiment applies a definition of the "effective filling time" that has the entrance of sample into the inlet as a starting point.

The filling of the reaction chamber 13 can be monitored using for example the already present frustrated total internal reflection (FTIR) detection method. Appropriate software in the evaluation module 53 can then monitor the fluid meniscus coming over different regions of interest (ROIs), distributed over the image. Using the software several ROIs can be defined over the probing area/sensor surface of the cartridge, in which observations can be made. During filling the meniscus of the fluid, which is filling the reaction chamber, runs over the defined ROIs and will give a change in optical signal. After being filled the signals are stable again. As the reaction chamber is filled in a given direction, e.g. from right to left, the rightmost ROI indicates the start of the filling, whereas the leftmost ROI indicates when the reaction chamber is completely filled.

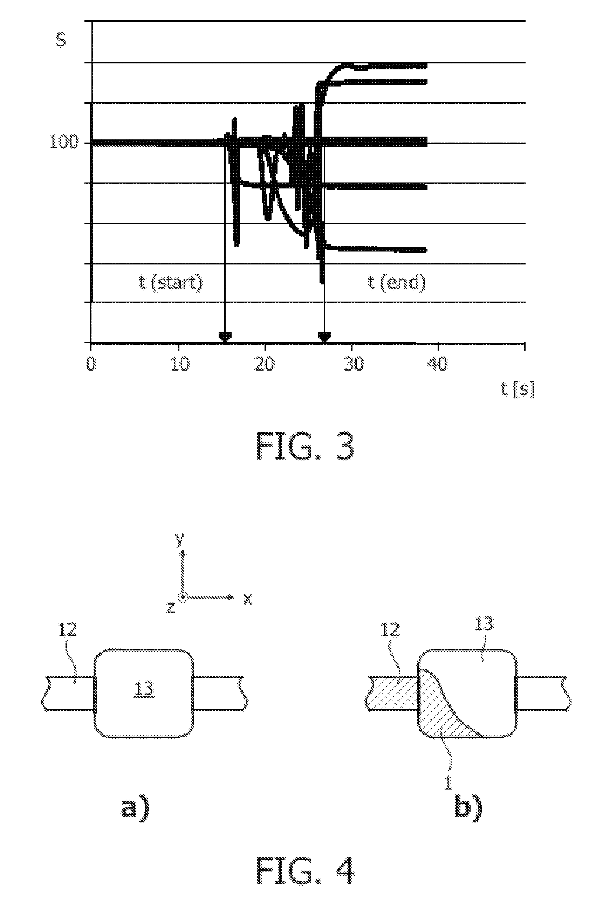

Figure 3 shows measured signal changes for all (ten) ROIs of an example in more detail. The graph shows a measurement starting with a stable optical signal, having big changes in optical signal during filling, and stable signals after filling. From this the starting time t_start and ending time t_end of the filling process can be determined. This embodiment applies a definition of the "effective filling time" that has the entrance of sample into the reaction chamber as a starting point.

Detection of the fluid in the reaction chambers 13 can also be performed by using wetting detector structures on the surface of the investigation region 14.

In another embodiment, detection of the fluid (meniscus) is performed by image processing algorithms to detect (in images provided by an image sensor 52) the shape of the liquid meniscus and to get a very accurate measurement of the portion of the reaction chamber filled for each time point. If dried beads 22 are located above the reaction chamber 13, the portion of the reaction chamber that is wetted (as detected in the FTIR image) is proportional to the beads that have already been redispersed and have started the reaction with the target molecules. This embodiment applies a definition of the "effective filling time" that has the entrance of sample into the reaction chamber as a starting point. Moreover, this embodiment can comprise the case that no agent is added at the inlet.

As sketched in Figure 4, the reaction chamber 13 will often not fill "straight". The aforementioned method provides nevertheless an accurate measure of the amount of beads that is already interacting with the sample fluid.

In general, a more elaborated embodiment of the invention will therefore use the filling-course of the reaction chamber (or even of the cartridge) with sample fluid instead of the single-valued "effective filling time". Observation of the fluid meniscus may for example yield that
- portion A of the agent has started redispersing at time t_{A},
- portion B of the agent has started redispersing at time t_{B},
- etc.
Different incubation times (t_{A}-t_end), (t_{B}-t_end), ... can be determined from this information for the portions A, B, ... of the agent, from which an "effective incubation" can be derived. This may for example be the average of the individual incubation times of the agent-portions (e.g. weighted with the amount/area of the portions).

In the following examples of wetting detection in a sensor device, the sensor surface of the reaction chamber is initially covered with a sucrose layer that is applied on top of printed antibodies to project the antibodies. Applying a fluid to such a sucrose layer will dissolve it.

As described above, proper wetting of a cartridge may be evaluated on the basis of a time-variant wetting profile imaged by FTIR. Said wetting profile may be analyzed by (1) subdividing an image of the sensor surface into multiple regions ("investigation regions" or "regions of interest"), and (2) checking the intensity change of each region over time. Via FTIR the sensor surface is imaged by a camera. Several regions of interest (ROIs) may be defined within the overall image to determine the signal change during the assay time. The relevant part of the assay starts after dissolution of the sucrose layer. During dissolution of the sucrose layer, a line indicating the sucrose dissolution moves over the image (similar to the fluid meniscus shown in Figure 4).

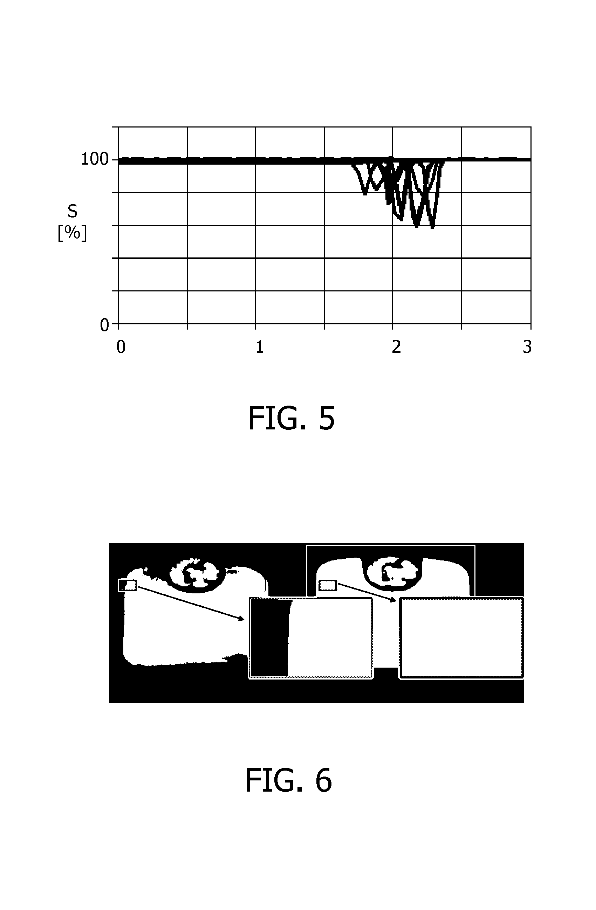

Figure 5 shows how the detected signal S of a region of interest will change as the aforementioned sucrose dissolving line passes the ROI. This (intermediate) signal change is hence an indicator of the presence of fluid in the detection chamber. The robustness of this detection can be increased by increasing the number of ROIs.

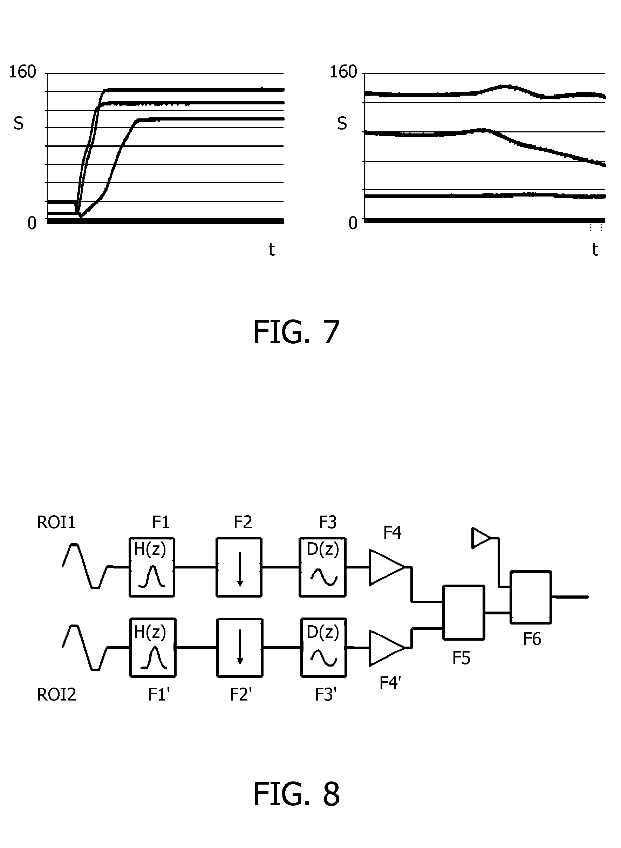

Figures 6 and 7 illustrate an embodiment in which regions of interest are defined on the FTIR image near the edges of the reaction chamber (only one such ROI is indicated and enlarged in Figure 6). The average intensity of the pixels inside such a ROI is monitored at 25 frames per second. This average intensity is the base signal to detect wetting.

During the process of wetting the monitored ROIs will change from dark to light due to the dissolution of the sucrose layer in the chamber. This can be seen from

Figure 6, in which the left images are before and the right images are after dissolution of the sucrose layer by wetting.

The diagrams of Figure 7 represent corresponding average image values S for several ROIs when wetting occurs (left diagram) and when no wetting occurs (right diagram), respectively. It can be seen that the dissolution of the sucrose layer causes a ramp in the signal (because the ROI is chosen on the border of the sample chamber, the signal level increases significantly after wetting due to the fact that the light area expands after dissolving of the sucrose layer). This ramp is used as the property to be detected to indicate wetting.

Apart from the signal caused by the dark to light transition, noise will usually be present due to cartridge movement. Cartridge movement can be caused by interaction of the operator with the cartridge for e.g. cartridge lid closure. Impact of noise on the signal can be minimized by placing the ROIs such that the maximal expected cartridge movement will not cause the ROIs to be positioned outside of the observation area. The remaining noise can be filtered out to prevent a false detection. Since wetting is inherently a slow process (multiple seconds), a low pass filter can be used to filter out the noise caused by - faster - cartridge movement. A two-step filter may be chosen to optimize the cutoff of the filter without causing a large processing overhead.

Figure 8 illustrates a possible design of filter for processing the pixel values of two regions of interest ROI1 and ROI2. This filtering system comprises:
- Anti-aliasing filters F 1 and F1' that prevent aliasing caused by the subsequent down sampling step.
- Down sampling filters F2 and F2' that reduce processing overhead.
- Differentiating low pass filters F3 and F3'. Differentiation is applied to reduce sensitivity to drift, while the low pass filters out noise due to cartridge movement.
- Elements F4 and F4' in which the maximal values are stored.

To further increase robustness of the wetting detection process, the results of multiple ROIs may be combined by averaging the maximum value for each ROI. This is done in unit F5. The resulting value is compared to a predefined threshold in unit F6. If it is above threshold, the output of unit F6 indicates wetting, and further analysis may be started.

While the invention was described above with reference to particular embodiments, various modifications and extensions are possible, for example:
- The sensor can be any suitable sensor to detect the presence of (magnetic) particles on or near to a sensor surface, based on any property of the particles, e.g. it can detect via magnetic methods (e.g. magnetoresistive, Hall, coils), optical methods (e.g. imaging, fluorescence, chemiluminescence, absorption, scattering, evanescent field techniques, surface plasmon resonance, Raman, etc.), sonic detection (e.g. surface acoustic wave, bulk acoustic wave, cantilever, quartz crystal etc), electrical detection (e.g. conduction, impedance, amperometric, redox cycling), combinations thereof, etc.
- In addition to molecular assays, also larger moieties can be detected with sensor devices according to the invention, e.g. cells, viruses, or fractions of cells or viruses, tissue extract, etc.
- The detection can occur with or without scanning of the sensor element with respect to the sensor surface.
- Measurement data can be derived as an end-point measurement, as well as by recording signals kinetically or intermittently.
- The particles serving as labels can be detected directly by the sensing method. As well, the particles can be further processed prior to detection. An example of further processing is that materials are added or that the (bio)chemical or physical properties of the label are modified to facilitate detection.
- The device and method can be used with several biochemical assay types, e.g. binding/unbinding assay, sandwich assay, competition assay, displacement assay, enzymatic assay, etc. It is especially suitable for DNA detection because large scale multiplexing is easily possible and different oligos can be spotted via ink-jet printing on a substrate.
- The device and method are suited for sensor multiplexing (i.e. the parallel use of different sensors and sensor surfaces), label multiplexing (i.e. the parallel use of different types of labels) and chamber multiplexing (i.e. the parallel use of different reaction chambers).
- The device and method can be used as rapid, robust, and easy to use point-of-care biosensors for small sample volumes. The reaction chamber can be a disposable item to be used with a compact reader, containing the one or more field generating means and one or more detection means. Also, the device, methods and systems of the present invention can be used in automated high-throughput testing. In this case, the reaction chamber is e.g. a well-plate or cuvette, fitting into an automated instrument.

## Claims

1. A method for the examination of a sample (1) in a cartridge (10) with an inlet (11) and a reaction chamber (13), said method comprising the following steps:
a) introducing the sample into the inlet (11);
b) applying at the inlet (11) an agent (21) to the sample;
c) letting the sample flow through a channel (12) from the inlet (11) to the reaction chamber (13);
d) measuring the filling-course of the cartridge (10) and/or the reaction chamber (13);
e) examining the sample in the reaction chamber (13), wherein the examination procedure and/or its evaluation are adapted according to the determined filling-course.

2. The method according to claim 1,
**characterized in that** the agent (21) comprises label particles that specifically bind to target components of the sample (1), particularly magnetic particles.

3. The method according to claim 1,
**characterized in that** the cartridge (10) comprises a filter (15) through which the sample (1) passes on its way from the inlet (11) to the reaction chamber (13).

4. The method according to claim 1,
**characterized in that** the adaptation of the evaluation comprises a correction of the examination results according to their dependence on the effective incubation of the sample with the agent.

5. The method according to claim 1,
**characterized in that** the adaptation of the examination procedure comprises an adaptation of its start and/or an adaptation of an actuation of the sample in the reaction chamber.

6. The method according to claim 1,
**characterized in that** the arrival of sample fluid (1) is detected at a plurality of investigation regions (13).

7. The method according to claim 1,
**characterized in that** the image data of a plurality of investigation regions (13) are processed.

8. An apparatus (100) for the examination of a sample (1), comprising
a) a cartridge (10) which comprises:
i) an inlet (11) where a sample can be introduced;
ii) a reaction chamber (13) where the sample can be examined;
iii) a channel (12) connecting the inlet (11) with the reaction chamber (13);
iv) a reservoir (16) for an agent (21) with which the sample shall be incubated, said reservoir being located at the inlet (11);
b) a filling detector (52, 53) for measuring the filling-course of the cartridge (10) and/or the reaction chamber (13); and
c) control and/or evaluation modules programmed to adapt the examination procedure and/or its evaluation according to the determined filling-course.

9. The apparatus (100) according to claim 8,
**characterized in that** the agent (21) comprises label particles that specifically bind to target components of the sample (1), particularly magnetic particles.

10. The apparatus (100) according to claim 8,
**characterized in that** the cartridge (10) comprises a filter (15) through which the sample (1) passes on its way from the inlet (11) to the reaction chamber (13).

11. The apparatus (100) according to claim 8,
**characterized in that** the effective incubation of the sample with an agent is determined from the filling-course, particularly from a measured spatiotemporal course of filling;
wherein, preferably, the adaptation of the evaluation comprises a correction of the examination results according to their dependence on the effective incubation of the sample with the agent..

12. The apparatus (100) according to claim 8,
**characterized in that** the filling detector comprises an imaging device (52) and an image processing device (53) for detecting a fluid meniscus in the reaction chamber (13).

13. The apparatus (100) according to claim 8,
**characterized in that** the arrival of sample fluid (1) is detected at a plurality of investigation regions (13).

14. The apparatus (100) according to claim 8,
**characterized in that** image data of a plurality of investigation regions (13) are processed.

15. The apparatus (100) according to claim 8,
**characterized in that** the reservoir (16) is pre-filled with the agent (21).

## Patentansprüche

1. Verfahren zur Untersuchung einer Probe (1) in einer Kartusche (10) mit einem Einlass (11) und einer Reaktionskammer (13), wobei das genannte Verfahren die folgenden Schritte umfasst:
a) Einführen der Probe in den Einlass (11);
b) Anwenden eines Mittels (21) am Einlass (11) auf die Probe;
c) Fließen lassen der Probe durch einen Kanal (12) von dem Einlass (11) zu der Reaktionskammer (13);
d) Messen des Füllverlaufs der Kartusche (10) und/oder der Reaktionskammer (13);
e) Untersuchen der Probe in der Reaktionskammer (13), wobei die Untersuchungsprozedur und/oder ihre Evaluierung entsprechend dem vorgegebenen Füllverlauf angepasst werden.

2. Verfahren nach Anspruch 1,
**dadurch gekennzeichnet, dass** das Mittel (21) Markerpartikel umfasst, die sich speziell an Zielkomponenten der Probe (1) binden, insbesondere magnetische Partikel.

3. Verfahren nach Anspruch 1,
**dadurch gekennzeichnet, dass** die Kartusche (10) einen Filter (15) umfasst, durch den die Probe (1) auf ihrem Weg vom Einlass (11) zur Reaktionskammer (13) fließt.

4. Verfahren nach Anspruch 1,
**dadurch gekennzeichnet, dass** die Anpassung der Evaluierung eine Korrektur der Untersuchungsergebnisse entsprechend ihrer Abhängigkeit von der effektiven Inkubation der Probe mit dem Mittel umfasst.

5. Verfahren nach Anspruch 1,
**dadurch gekennzeichnet, dass** die Anpassung der Untersuchungsprozedur eine Anpassung ihres Beginns und/oder eine Anpassung einer Betätigung der Probe in der Reaktionskammer umfasst.

6. Verfahren nach Anspruch 1,
**dadurch gekennzeichnet, dass** die Ankunft des Probenfluids (1) bei einer Vielzahl von interessierenden Regionen (13) detektiert wird.

7. Verfahren nach Anspruch 1,
**dadurch gekennzeichnet, dass** die Bilddaten einer Vielzahl von interessierenden Regionen (13) verarbeitet werden.

8. Gerät (100) zur Untersuchung einer Probe (1), umfassend:
a) eine Kartusche (10), die Folgendes umfasst:
i) einen Einlass (11), in den eine Probe eingeführt werden kann;
ii) eine Reaktionskammer (13), in der die Probe untersucht werden kann;
iii) einen Kanal (12), der den Einlass (11) mit der Reaktionskammer (13) verbindet;
iv) ein Reservoir (16) für ein Mittel (21), mit dem die Probe inkubiert werden soll, wobei sich das genannte Reservoir am Einlass (11) befindet;
b) einen Füllungsdetektor (52, 53) zum Messen des Füllverlaufs der Kartusche (10) und/oder der Reaktionskammer (13); und
c) Steuer- und/oder Evaluierungsmodule, die programmiert sind, um die Untersuchungsprozedur und/oder ihre Evaluierung entsprechend dem ermittelten Füllverlauf anzupassen.

9. Gerät (100) nach Anspruch 8,
**dadurch gekennzeichnet, dass** das Mittel (21) Markerpartikel umfasst, die sich speziell an Zielkomponenten der Probe (1) binden, insbesondere magnetische Partikel.

10. Gerät (100) nach Anspruch 8,
**dadurch gekennzeichnet, dass** die Kartusche (10) einen Filter (15) umfasst, durch den die Probe (1) auf ihrem Weg vom Einlass (11) zur Reaktionskammer (13) fließt.

11. Gerät (100) nach Anspruch 8,
**dadurch gekennzeichnet, dass** die effektive Inkubation der Probe mit dem Mittel anhand des Füllverlaufs ermittelt wird, insbesondere anhand eines gemessenen räumlich-zeitlichen Verlaufs des Füllvorgangs;
wobei vorzugsweise die Anpassung der Evaluierung eine Korrektur der Untersuchungsergebnisse entsprechend ihrer Abhängigkeit von der effektiven Inkubation der Probe mit dem Mittel umfasst.

12. Gerät (100) nach Anspruch 8,
**dadurch gekennzeichnet, dass** der Fülldetektor eine Bildgebungsvorrichtung (52) und eine Bildverarbeitungsvorrichtung (53) zum Detektieren eines Fluidmeniskus in der Reaktionskammer (13) umfasst.

13. Gerät (100) nach Anspruch 8,
**dadurch gekennzeichnet, dass** die Ankunft des Probenfluids (1) bei einer Vielzahl von interessierenden Regionen (13) detektiert wird.

14. Gerät (100) nach Anspruch 8,
**dadurch gekennzeichnet, dass** die Bilddaten einer Vielzahl von interessierenden Regionen (13) verarbeitet werden.

15. Gerät (100) nach Anspruch 8,
**dadurch gekennzeichnet, dass** das Reservoir (16) mit dem Mittel (21) vorgefüllt ist.

## Revendications

1. Procédé d'examen d'un échantillon (1) dans une cartouche (10) avec une entrée (11) et une chambre réactionnelle (13), ledit procédé comprenant les étapes suivantes consistant à :
a) introduire l'échantillon dans l'entrée (11) ;
b) appliquer à l'entrée (11) un agent (21) à l'échantillon ;
c) laisser l'échantillon s'écouler à travers un canal (12) de l'entrée (11) à la chambre réactionnelle (13) ;
d) mesurer le cours de remplissage de la cartouche (10) et/ou de la chambre réactionnelle (13) ;
e) examiner l'échantillon dans la chambre réactionnelle (13), dans lequel la procédure d'examen et/ou son évaluation est ou sont adaptées selon le cours de remplissage déterminé.

2. Procédé selon la revendication 1,
**caractérisé en ce que** l'agent (21) comprend des particules de marquage qui se lient spécifiquement à des composants cibles de l'échantillon (1), en particulier des particules magnétiques.

3. Procédé selon la revendication 1,
**caractérisé en ce que** la cartouche (10) comprend un filtre (15) à travers lequel l'échantillon (1) passe sur son trajet de l'entrée (11) à la chambre réactionnelle (13).

4. Procédé selon la revendication 1,
**caractérisé en ce que** l'adaptation de l'évaluation comprend une correction des résultats d'examen en fonction de leur dépendance à l'incubation efficace de l'échantillon avec l'agent.

5. Procédé selon la revendication 1,
**caractérisé en ce que** l'adaptation de la procédure d'examen comprend une adaptation de son départ et/ou une adaptation d'une manipulation de l'échantillon dans la chambre réactionnelle.

6. Procédé selon la revendication 1,
**caractérisé en ce que** l'arrivée d'un fluide échantillon (1) est détectée dans une pluralité de régions d'investigation (13).

7. Procédé selon la revendication 1,
**caractérisé en ce que** les données d'image d'une pluralité de régions d'investigation (13) sont traitées.

8. Appareil (100) pour l'examen d'un échantillon (1), comprenant :
a) une cartouche (10) qui comprend :
i) une entrée (11) où un échantillon peut être introduit ;
ii) une chambre réactionnelle (13) où l'échantillon peut être examiné ;
iii) un canal (12) raccordant l'entrée (11) à la chambre réactionnelle (13) ;
iv) un réservoir (16) pour un agent (21) avec lequel l'échantillon doit être incubé, ledit réservoir étant situé à l'entrée (11) ;
b) un détecteur de remplissage (52, 53) pour mesurer le cours de remplissage de la cartouche (10) et/ou de la chambre réactionnelle (13) ; et
c) des modules de commande et/ou d'évaluation programmés pour adapter la procédure d'examen et/ou son évaluation en fonction du cours de remplissage déterminé.

9. Appareil (100) selon la revendication 8,
**caractérisé en ce que** l'agent (21) comprend des particules de marquage qui se lient spécifiquement à des composants cibles de l'échantillon (1), en particulier des particules magnétiques.

10. Appareil (100) selon la revendication 8,
**caractérisé en ce que** la cartouche (10) comprend un filtre (15) à travers lequel l'échantillon (1) passe sur son trajet de l'entrée (11) à la chambre réactionnelle (13).

11. Appareil (100) selon la revendication 8,
**caractérisé en ce que** l'incubation efficace de l'échantillon avec un agent est déterminée à partir du cours de remplissage, en particulier d'un cours de remplissage spatiotemporel mesuré ;
dans lequel, de préférence, l'adaptation de l'évaluation comprend une correction des résultats d'examen en fonction de leur dépendance à l'incubation efficace de l'échantillon avec l'agent.

12. Appareil (100) selon la revendication 8,
**caractérisé en ce que** le détecteur de remplissage comprend un dispositif d'imagerie (52) et un dispositif de traitement d'image (53) pour détecter un ménisque de fluide dans la chambre réactionnelle (13).

13. Appareil (100) selon la revendication 8,
**caractérisé en ce que** l'arrivée d'un fluide échantillon (1) est détectée dans une pluralité de régions d'investigation (13).

14. Appareil (100) selon la revendication 8,
**caractérisé en ce que** les données d'image d'une pluralité de régions d'investigation (13) sont traitées.

15. Appareil (100) selon la revendication 8,
**caractérisé en ce que** le réservoir (16) est pré-rempli par l'agent (21).
